(19) **Europäisches Patentamt**
**European Patent Office**
**Office européen des brevets**

(11) **EP 1 682 167 B1**

(12) **EUROPÄISCHE PATENTSCHRIFT**

(45) Veröffentlichungstag und Bekanntmachung des Hinweises auf die Patenterteilung:
**18.02.2009 Patentblatt 2009/08**

(51) Int Cl.:
***A61K 38/42*** *(2006.01)*    ***A61P 7/10*** *(2006.01)*
***A61P 11/00*** *(2006.01)*

(21) Anmeldenummer: **04797509.9**

(22) Anmeldetag: **02.11.2004**

(86) Internationale Anmeldenummer:
**PCT/EP2004/012363**

(87) Internationale Veröffentlichungsnummer:
**WO 2005/046717 (26.05.2005 Gazette 2005/21)**

(54) **VERWENDUNG HYPERPOLYMERER HÄMOGLOBINE ZUR BEHANDLUNG EINES LUNGENÖDEMS**

USE OF HYPERPOLYMER HAEMOGLOBIN FOR TREATING A PULMONARY OEDEMA

UTILISATION D'HEMOGLOBINE HYPERPOLYMERE POUR TRAITER UN OEDEME PULMONAIRE

(84) Benannte Vertragsstaaten:
**AT BE BG CH CY CZ DE DK EE ES FI FR GB GR HU IE IS IT LI LU MC NL PL PT RO SE SI SK TR**

(30) Priorität: **12.11.2003 DE 10352692**

(43) Veröffentlichungstag der Anmeldung:
**26.07.2006 Patentblatt 2006/30**

(73) Patentinhaber: **SanguiBioTech GmbH**
**58455 Witten (DE)**

(72) Erfinder: **BARNIKOL, Wolfgang**
**55128 Mainz (DE)**

(74) Vertreter: **Müller, Claudia**
**European Patent Attorney**
**Uhlandstrasse 58**
**60314 Frankfurt / Main (DE)**

(56) Entgegenhaltungen:
**WO-A-02/00230**      **WO-A-02/00768**
**WO-A-03/094953**

- **BARNIKOL ET AL: "Hyperpolymere Hämoglobine als künstliche Sauerstoffträger" THERAPIEWOCHE, Bd. 15, 1996, Seiten 811-815, XP009044173 in der Anmeldung erwähnt**
- **BARNIKOL WOLFGANG K R ET AL: "New artificial oxygen carriers made of pegulated polymerised pyridoxylated porcine haemoglobin (P4Hb)" COMPARATIVE BIOCHEMISTRY AND PHYSIOLOGY PART A MOLECULAR AND INTEGRATIVE PHYSIOLOGY, Bd. 132A, Nr. 1, Mai 2002 (2002-05), Seiten 185-191, XP002318526 ISSN: 1095-6433**
- **VANDEGRIFF K D: "HAEMOGLOBIN-BASED OXYGEN CARRIERS" EXPERT OPINION ON INVESTIGATIONAL DRUGS, ASHLEY PUBLICATIONS LTD., LONDON, GB, Bd. 9, Nr. 9, 2000, Seiten 1967-1984, XP008034792 ISSN: 1354-3784 in der Anmeldung erwähnt**
- **BARNIKOL W K R ET AL: "Haemoglobin hyperpolymers, a new type of artificial oxygen carrier - Concept and current state of development" TRANSFUSION MEDICINE AND HEMOTHERAPY, Bd. 31, Nr. 4, August 2004 (2004-08), Seiten 269-281, XP009044191 ISSN: 1660-3796**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Gegenstand vorliegender Erfindung

Die vorliegende Erfindung betrifft die Verwendung hypo-onkotischer, wässriger Lösungen molekular-disperser chemisch modifizierter, hoch-molekular vernetzter Hämoglobine, sogenannter Hämoglobin-Hyperpolymere, zur Herstellung von Mitteln, zur symptomatischen, primär lebensrettenden Behandlung akuter Lungenödeme. Die Verabreichung erfolgt insbesondere intravasal. Überraschender Weise kann eine additive Verabreichung vorgenommen werden, da erfindungsgemäß der kolloid-osmotische (= onkotische) Druck des Blutes nur wenig erhöht und das Blutvolumen deshalb kaum vergrößert wird. Die erfindungsgemäße Anwendung bzw. Verabreichung ist somit (fast) Volumenneutral bezogen auf das Blut, in das die Injizierung vorgenommen wird. Somit wird erstmals ein hyperpolymeres Hämoglobin-Derivat therapeutisch als Blut-Additiv zur Behandlung eines Lungenödems eingesetzt.

Hintergrund der Erfindung

1. Künstliche Sauerstoffträger

Künstliche Sauerstoffträger / -transporter sind eine äußerst heterogene Gruppe von Stoffen. Ihre namensgebenden Charakteristika sind ihre Fähigkeit, Sauerstoff in Form des molekularen Disauerstoffes ($O_2$) reversibel zu binden oder in sich zu lösen - damit haben sie eine prinzipielle Eigenschaft mit dem natürlichen Sauerstoffträger / -transporter im Blut, dem Hämoglobin (Roter Blutfarbstoff), das in den Erythrozyten (Roten Blutzellen) vorkommt, gemein - sowie ihr potentielle Verwendbarkeit als intravasal (i. d. R. also intravenös) zu applizierende Pharmazeutika oder in sonstigen biomedizinischen Anwendungen.

(Eine umfassende Übersicht (Stand der Technik) in: RIESS J. G.: "Oxygen Carriers ("Blood Substitutes") - Raison d'Etre, Chemistry, and Some Physiology", Chemical Reviews 101 (2001): 2797 - 2919; eine Übersicht zu vielen Hämoglobin-Derivaten in: VANDEGRIFF K. D.: "Haemoglobin-based Oxygen Carriers", Expert Opinions on Investigational Drugs 9 (2000): 1967 - 1984) Die bekannten Sauerstoffträger unterscheiden sich sowohl hinsichtlich ihrer Beschaffenheit als auch der dadurch bedingten physikochemischen Eigenschaften und ihrer Verwendbarkeit.

So sind Perfluorocarbone mit und in wässrigen Lösungen, wie bspw. dem Blutplasma, nicht mischbar bzw. darin nicht lösbar. Sie können aber in Form fein disperser (mittels Emulgatoren stabilisierter) Tröpfchen darin emulgiert werden. Ebenfalls emulgiert oder suspendiert werden mit natürlichen oder künstlichen Sauerstoffträgern gefüllte Liposomen. Hierbei handelt es sich um mit einer Phospholipid-Doppelschicht-Membran umgebene Vesikel (künstliche Zellen oder auch künstliche Erythrozyten).

Frei in der wässrigen Phase (bspw. im Plasma) gelöst werden können Hämoglobine, ihre durch chemische Modifikation erhältlichen Derivate, sowie isolierte und notwendigerweise chemisch modifizierte Häm-Gruppen.

Der molekulare strukturelle Aufbau künstlicher Sauerstoffträger bedingt deren Art der Verabreichung, insbesondere, ob sie als ein <u>Ersatz</u> fehlendes Blut substituieren können, oder ob sie in vorhandenes Blut als <u>Zusatz</u> addiert werden können. Bisher beschriebene Produkte haben einen Sauerstofftransportierenden Plasma ersatz zum Ziel, also eine Plasmaersatz-Flüssigkeit zum Auffüllen des durch eine akute Blutung oder Blutentnahme teil-entleerten Blutgefäßsystems, die gegenüber den bekannten (nicht Sauerstoff transportierenden) Plasmaersatzmitteln darüber hinaus auch eine weitere wesentliche Funktion des Blutes, nämlich die des Sauerstofftransports, restituiert.

Perfluorocarbone und Liposomen lösen sich nicht im wässrigen Blutplasma, sie besitzen und erfüllen als deutlich getrennte, eigene, emulgierte oder suspendierte Phase ein gewisses Volumen, und erscheinen deshalb zum genannten Zweck als Sauerstoff-transportierender Plasmaersatzmittel prinzipiell geeignet, nicht dagegen als ein Zusatz in das Blut, da sie dessen Volumen zwangsläufig vergrößern.

Um als Ersatz fehlenden Blutes geeignet zu sein, müssen Sauerstoff-transportierende Plasmaersatzmittel aus frei in einer wässrigen Phase gelösten Hämoglobinen, oder ihren durch chemische Modifikationen erhaltenen Derivaten, sowohl in etwa isoton (die Tonizität ist ein relatives Maß für den osmotischen Druck) als auch isonkotisch (= iso-onkotisch, die Onkosie ist ein Maß für den onkotischen (= kolloid-osmotischen) Druck) mit dem Blutplasma sein. Zur Erzielung einer Isotonie befinden sich solche künstlichen Sauerstoffträger i. d. R. gelöst in einer Elektrolytlösung, die dem Blutplasma-Elektrolyten ähnelt.

Iso-Onkosie in pharmazeutischen Zubereitungen bisher entwickelter (und publizierter) Hämoglobin-Derivate als künstliche Sauerstoffträger bedingen diese selbst. Ihr molekulares Design entspricht der klinischen Anforderung nach einer Isonkosie, die durch eine hinreichende Anzahl onkotisch wirksamer Wirkstoffmoleküle realisiert wird.

Daher werden derartige, frei gelöste Hämoglobinderivate auch ganz besonders zur Anwendung bei (stärkeren) Blutverlusten vorgeschlagen. Für medizinische Indikationen ohne einen Blutverlust sind sie nur sehr bedingt (nämlich von der Menge/Dosis her äußerst begrenzt) einsetzbar, da sie aufgrund ihrer genannten Eigenschaften das Blutvolumen zwingend um das Volumen ihrer injizierten oder infundierten pharmazeutischen Zubereitung vergrößern.

2. Hämoglobin-Hyperpolymere

Sollen künstliche Sauerstoffträger zur Behandlung eines Sauerstoffmangels als Additive eingesetzt werden, so sollten diese einen ausreichend geringen kolloid osmotischen Druck aufweisen (vgl. Barnikol W. K. R., et al. (1996): "Hyperpolymere Hämoglobine als künstliche Sauerstoffträger - Ein innovativer Ansatz der medizinischen Entwicklung", Therapiewoche 46: 811 - 815). Sie sind konzipiert, um als künstliche Sauerstoffträger die Sauerstoff-Transportkapazität vorhandenen Blutes zu erhöhten, wenn kein Blutverlust zu ersetzen ist. Damit Hämoglobin-Hyperpolymere nach Injektion oder Infusion das Volumen des zirkulierenden Blutes nicht bleibend vergrößern (sondern vielmehr das Wasser und die Salze ihrer Zubereitung möglichst umfänglich über die Nieren wieder ausgeschieden werden), muss die onkotisch wirksame Anzahl an Wirkstoffmolekülen so weit wie möglich verringert werden. Dazu werden die Hämoglobine chemisch (mittels poly- oder bifunktioneller Vernetzer) quer-vernetzt und polymerisiert. So entstehen künstliche, Sauerstoff bindende Riesenmoleküle. Chemisch betrachtet sind molekular vernetzte Hämoglobine Multimere des Monomeren Hämoglobin. Dabei wird jedoch keinerlei Aussage darüber getroffen, welche Multimere - es handelt sich hierbei um eine breite Molekulargewichtsverteilung mit Oligomeren und höheren Polymeren - welche Auswirkungen auf die Eigenschaften des Gesamtproduktes haben.

3. Lungenödeme

Ein Ödem ist eine abnorme Flüssigkeitsansammlung im Interzellularraum (Interstitium). Lungenödeme sind ein häufiges klinisches Krankheitsbild. Sie führen zu einer lebensbedrohlichen Beeinträchtigung der Gesundheit, die in schweren Fällen tödlich verläuft. Man unterscheidet hauptsächlich das durch Insuffizienz des linken Ventrikels bedingte kardiale (Stauungs-) Ödem und die toxisch bedingten Lungenödeme mit Erhöhung der Kapillarpermeabilität bei Lungenentzündungen, Inhalation schädigender Gase, bspw. auch bei hohen Sauerstoff Konzentrationen, Urämie, oder bei Überempfindlichkeitsreaktionen etc.

Die Therapie ist immer symptomatisch bezüglich der lebensbedrohlichen Beeinträchtigung der Lungenfunktion (Intensivmedizinische Versorgung, Kortikoide zur Unterdrückung entzündlicher Vorgänge, Sauerstoff-angereicherte Atemluft und Überdruckbeatmung, etc.) und wenn möglich kausal bezüglich der Ursachen (Expositionsprophylaxe, Therapie der kardialen Insuffizienz oder der zugrunde liegenden Nierenkrankheit, etc.)

(Zum Stand der Technik siehe bspw.: Böcker, W, Denk, H. Heitz Ph. U (Hrsg): Pathologie, Urban & Schwarzenberg, München u. a. 1997; Gerock W. Huber CH, Meinertz T, Zeidler H (Hrsg.): Gross = Schölmerich = Gerock - Die Innere Medizin, 10. völlig neu bearb. und erw. Auflage, Schattauer, Stuttgart und New York 2000; Weikrauch T.R.. (Hrsg.): Wolff = Weikrauch - Internistische Therapie 2000/2001, 13. neubearb. Auflage, Urban & Fischer, München und Jena 2000; Arch. Cardiol. Mex., Vol. 72, Seite 280-285).

Aufgabe der Erfindung

Der vorliegenden Erfindung liegt die Aufgabe zugrunde, eine verbesserte symptomatische Therapie des akuten Lungenödems, insbesondere der hohen Letalität dieser Erkrankungen (klinisch werden Sterblichkeiten zwischen 30 und 90 % angegeben), bereit zu stellen.

Lösung der Aufgabe

Diese Aufgabe wird erfindungsgemäß gelöst durch die Herstellung und Anwendung einer hypo-onkotischen Lösung chemisch modifizierter, hochmolekular vernetzter hyperpolymerer Hämoglobine. Überraschender Weise kann mit solchen Lösungen wirksam ein akutes Lungenödem therapiert und die Sterblichkeit verringert werden.

Nähere Beschreibung der Erfindung

Nach vorliegender Erfindung kann ein akutes Lungenödem wirksam behandelt werden durch die Verabreichung einer wässrigen Lösung in das Blut addierbarer hyperpolymerer Hämoglobin-Derivate, deren onkotischer Druck in wässriger Lösung viel geringer ist als der des vorhandenen Blutes, und somit als Additiv einen hypo-onkotischen Druck zeigt.

Beschreibung der Abbildungen

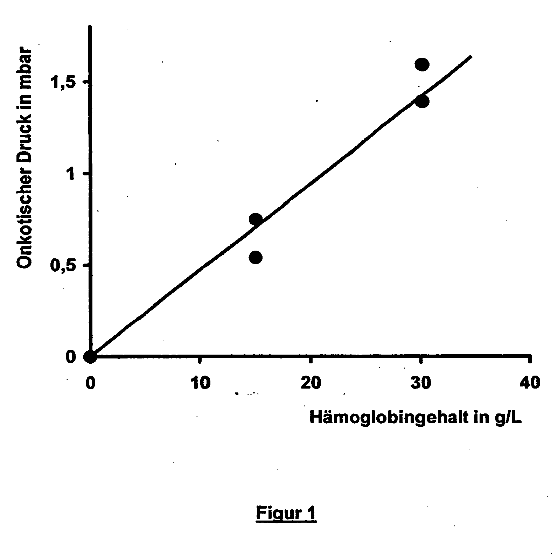

Figur 1 zeigt beispielhaft die Abhängigkeit des onkotischen Druckes ($\pi_{onk}$) einer Lösung eines erfindungsgemäß zur verbesserten Therapie eines akuten Lungenödem verwendeten chemisch modifizierten, hoch-molekular vernetzten Hämoglobins.

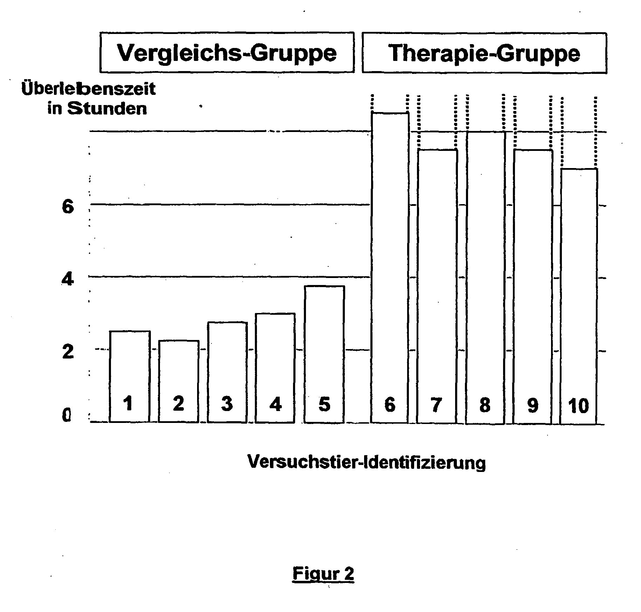

Figur 2 zeigt die Wirksamkeit chemisch modifizierten, hoch-molekular vernetzten Hämoglobins zur verbesserten Therapie eines akuten Lungenödem, hier als die Überlebenszeit von zehn narkotisierten Ratten nach Beibringung eines letalen toxischen Lungenödems (durch Injektion von Ölsäure), von denen fünf Tiere zur Therapie den Wirkstoff in ihr Blut addiert bekamen.

Besondere Ausführungsform der Erfindung

Gemäß der Erfindung werden Mittel bereitgestellt und verwendet, nämlich wässrige Lösungen, enthaltend die genannten hyperpolymeren Hämoglobine. Insbesondere sind die wässrigen Lösungen Elektrolyt-haltige Lösungen. Vor allem entsprechen diese dem physiologischen Milieu. Die erfindungsgemäß eingesetzten chemisch modifizierten Sauerstoffträger (hyperpolymere Hämoglobine) stammen vom Menschen, vom Schwein oder vom Rind. Bevorzugt stammen sie vom Schwein.

Die erfindungsgemäß verwendeten hyperpolymeren Hämoglobine sind hoch molekulare, intermolekular vernetzte Hämoglobine. Die intermolekulare Vernetzung von Hämoglobinen ist allgemein bekannt und z. B. beschrieben in DE 197 01 037, EP 97 100790, DE 44 18 973, DE 38 41 105, DE 37 14 351, DE 35 76 651. Diese bekannten Verfahren sind daher hier inkorporiert.

Die Hämoglobin-Hyperpolymeren können, außer einer intermolekularen Vernetzung (Polymerisation), vielfältig weiter chemisch modifiziert sein. Beispielsweise können zur Modifikation der Affinität und Kooperativität der Ligandenbindung chemisch reaktive Effektoren kovalent angeknüpft sein. Für verschiedene gewünschte funktionelle Verbesserungen der Hämoglobin-Hyperpolymere, wie bspw. eine Verminderung ihrer Immunogenität oder eine Verlängerung der Verweildauer im Gefäßsystem (Katren, V.: "The Conjugation of Proteins With Polyethylene Glycol and Other Polymers - Altering Properties of Proteins to Enhance Their Therapeutic Potential", Advanced Drug Delivery Reviews 10 (1993): 91 - 114), oder eine Verbesserung der Verträglichkeit mit Proteinen des 'Empfänger'-Blutplasmas (DE 100 31 744 A1) können weitere Makromoleküle (wie bspw. Polyethylenoxide, Polyethylenglykole, Dextrane, Hydroxyethylstärken, etc.) unterschiedlicher Kettenlängen (Molmassen) kovalent angeknüpft sein.

In einer bevorzugten Ausführungsform ist an die modifizierten hyperpolymeren Hämoglobine ein Makromolekül, insbesondere ein Polyalkylenoxid, kovalent gebunden.

Eine besonders bevorzugte Ausgestaltung der Erfindung verwendet Hämoglobin-Hyperpolymere, die gemäß den deutschen Patentanmeldungen DE (OS) 100 31 740, DE (OS) 100 31 742 und DE (OS) 100 31 744 A1 hergestellt werden, deren Inhalt hier inkorporiert ist. Es handelt hierbei um polymerisierte Produkte (intermolekulare Vernetzung), wobei weiterhin noch eine Pegylierung (kovalente Verknüpfung mit Polyalkylenoxiden) vorgenommen ist.

In einer weiteren bevorzugten Ausführungsform kann gewünschtenfalls zusätzlich hierzu noch eine Umsetzung mit chemisch reaktiven Effektoren wie Pyridoxal-5'-Phosphat oder 2-Nor-2-Formyl-Pyridoxal-5'-Phosphat (intramolekulare Vernetzung), oder die Reaktion kann auch in Anwesenheit von chemisch nicht reaktiven Effektoren der Sauerstoffbindung wie z. B. 2,3-Bisphosphoglycerat, Inositolhexaphosphat, Inositolhexasulfat oder Mellitsäure stattfinden oder es kann eine Kombination dieser Umsetzung bzw. Milieubedingung vorgenommen sein. Derartige Produkte sind bekannt und wie oben erläutert beschrieben.

Bevorzugt sind solche Sauerstoffträger, die polymerisiert sind zum Beispiel mit für die intermolekulare Umsetzung bekannten bifunktionellen Vernetzer wie Butadiendiepoxid, Divinylsulfon, Diisocyanat, insbesondere Hexamethylendiisocyanat, Zyklohexyldiisocyanat und 2,5-Bisisocyanatobenzolsulfonsäure, Di-N-Hydroxysuccinimidylester, Diimidoester, oder Dialdehyd, insbesondere Glyoxal, der analog reagierende Glykolaldehyd, oder Glutardialdehyd. Diese Produkte werden dann insbesondere mit einem Polyethylenglykol oder anderen geeigneten Makromolekülen verknüpft. Hierzu gehören beispielsweise Polyethylenoxid, Polypropylenoxid, oder ein Copolymer aus Ethylenoxid und Propylenoxid oder ein Ester, Ether oder Esteramid hiervon. Es ist ferner bevorzugt, wenn das kovalent angeknüpfte Polyalkylenoxid eine Molare Masse von 200 bis 5000 g/mol aufweist.

Die Herstellung solchermaßen modifizierter Sauerstoffbinder ist in den oben genannten deutschen Patentanmeldungen beschrieben und hierin inkorporiert.

Ganz besonders bevorzugt sind Hyperpolymere, die aus desoxygeniertem Schweinehämoglobin, mit Glutardialdehyd als bifunktionellem Vernetzer und Polyethylenglykol als kovalent gebundenem Makromolekül zur Oberflächenmodifikation hergestellt sind, siehe DE 100 31 740 A1 oder DE 100 31 744 A1.

Erfindungsgemäß hat sich gezeigt, dass Hämoglobin-Hyperpolymere mit einem (mittleren) Polymerisationsgrad, der hinreichend groß ist, dass sie als künstliche Sauerstoffträger als ein therapeutisches Blut-Additiv (also ohne das Blutvolumen mehr als gering zu vergrößern, s. o.) in das Blut hinein-gebracht werden können, geeignet sind, wenn sie in einer wässrigen Elektrolytlösung nur einen gewissen, niedrigen onkotischen Druck erzeugen. Dieser steht in Beziehung mit dem geeigneten Mittelwert des Polymerisationsgrades (oder der proportionalen Molmasse) des modifizierten polymeren Hämoglobins. Hierbei handelt es sich um das Zahlenmittel, weil die Anzahl der wirksamen Moleküle für den onkotischen Druck verantwortlich ist.

Insbesondere hat sich gezeigt, dass die genannten Hyperpolymeren dann geeignet sind, wenn ein Polymerisationsgrad vorhanden ist, der hinreichend groß ist, dass der onkotische Druck von Lösungen mit den therapeutischen Konzentrationen der chemisch modifizierten, hochmolekular vernetzten Hämoglobine in einem wässrigen Elektrolyt-haltigen Milieu, (ohne sonstige

Makromoleküle) weniger als 5 mbar beträgt. Dies sind etwa 1/7 (und somit weniger als 15 %) des onkotischen Druckes menschlichen Blutplasmas von etwa 35 mbar (Applikationen der Mengen der Hämoglobin-Hyperpolymere, die im Blutplasma die genannten therapeutischen Konzentrationen ergeben, führen also zu Zunahmen des Blutplasmavolumens von maximal etwa 15 %).

Für ideale Lösungen kann der onkotische Druck ($\pi_{onk}$) aus der Molaren Masse (M) und dem Gehalt (als Massenkonzentration $c_m$) des gelösten Kolloids, sowie der allgemeinen Gaskonstanten (R) und der absoluten Temperatur (T) errechnet werden gemäß

$$\pi_{onk} \quad = \quad c_m \cdot R \cdot T \cdot M^{-1} \quad .$$

Für einen wie beschrieben festgelegten oberen Grenzwert des onkotischen Druckes ($\pi_{onk}$) von 5 mbar für ein Blut Additiv errechnet sich aus einer gewünschten therapeutischen Konzentration ($c_m$) im Blutplasma nach dieser Formel eine minimale Molmasse (als Zahlenmittel) der Hämoglobin-Hyperpolymere von M = (4.910 L/mol) - $c_m$, für 2 mbar von M = (12.300 L/mol) - $c_m$.

Reale Lösungen zeigen aber eine mit der Konzentration des Kolloids zunehmende Abweichung der onkotischen Drücke zu größeren Werten. Die genannte Formel für ideale Lösungen kann also bestenfalls zur Abschätzung der minimalen Molmassen dienen, für real existierende Polymere muss der reale onkotische Druck experimentell ermittelt werden, zumal er in nicht vorhersagbarer Weise vom strukturellen Aufbau der Polymeren abhängt. Beispielsweise errechnet man (die folgenden Werte sind der experimentell ermittelten Kurve des onkotischen Druckes über der Massenkonzenztration, gezeigt als Figur 1, entnommen) für die in den Beispielen verwendete Charge MR A-A aus einer Konzentration von 20 g/L und einem onkotischen Druck von etwa 1 mbar eine zugehörige (ideale) Molmasse von 491.000 g/mol, während die experimentell ermittelte reale Molmasse nur 320.000 g/mol beträgt.

Ganz besonders bevorzugt sind modifizierte Hämoglobine der beschriebenen Art, deren wässrige Elektrolyt-Lösungen einen onkotischen Druck von weniger als 2 mbar, zeigen.

Als wässrige Elektrolytlösungen für die erfindungsgemäße Anwendung der Hämoglobin-Hyperpolymeren sind sämtliche Lösungen mit Zusammensetzungen an Salzen geeignet, die das Extrazellularmilieu des Menschen (einschließlich des physiologischen pH-Wertes meist um 7,4 (zwischen 7,1 und 7,6)) imitieren oder diesem ähneln, somit insbesondere auch alle Vollelektrolyt-Infusionslösungen zur Elektrolytzufuhr und Kreislaufunterstützung (Übersicht in: Rote Liste Service GmbH (Hrsg.): Rote Liste 2002 - Arzneimittelverzeichnis für Deutschland (einschließlich EU-Zulassungen und bestimmter Medizinprodukte), ECV, Aulendorf 2002 (Kapitel 52, "52. Infusions- und Standard-Injektionslösungen, Organperfusionslösungen"). Diese sind bekannt. Insbesondere bevorzugt sind wässrige Elektrolytlösungen, enthaltend Wasser und Kochsalz in einer Konzentration zwischen 50 und 150 g/L, vor allem 70 bis 100 g/L.

Anwendung

Somit ist es überraschender Weise möglich, die Schwere eines akuten Lungenödems klinisch zu bessern, und zwar vor allem durch intravasal verabreichte chemisch modifizierte, hoch-molekular vernetzte hyperpolymere Hämoglobine als ein Blut-Additiv, fast ohne das Blutvolumen des Patienten zu vergrößern. Dabei war nicht zu erwarten, dass derartige Sauerstoffträger, wenn sie die beschriebenen Eigenschaften aufweisen, als Additiv so eingesetzt werden können, da diese chemisch modifizierten, hoch-molekular vernetzten Hämoglobine eigentlich als künstliche Sauerstoffträger entwickelt wurden und werden mit der Zielsetzung, periphere Gewebe mit Sauerstoff zu versorgen. Völlig überraschend war daher ihre Wirksamkeit zur Verbesserung der Therapie akuter Lungenödeme. Die (prä-)klinische Verbesserung zeigte sich in einem verbesserten Überleben, also einer verringerten Mortalität, in einem Tiermodell (der narkotisierten Ratte) experimenteller toxischer Lungenödeme, von denen nachfolgend Beispiele angeführt werden.

Die Verabreichung des Sauerstoffträgers erfolgt derart, dass die therapeutischen Konzentrationen im Blutplasma aus Gründen der zunehmenden Viskosität des Blutplasmas nicht wesentlich größer sind als 50 g/L, z. B. 50 bis 60 g/L, und insbesondere zwischen 10 und 40 g/L liegen. Andererseits können auch schon sehr geringe Konzentrationen (Bspw. ab 1 g/L) für eine Therapie ausreichend sein.

Der Sauerstoffträger kann in Konzentrationen von 20 bis 200 g /L, insbesondere 50 bis 100 g /L in der wässrigen Lösung vorliegen.

Das Mittel kann je nach Bedarf als einmalige Gabe oder auch als periodische oder unregelmäßige wiederholte Gabe verabreicht werden, wobei sich die Art und Menge nach dem Zustand, dem Alter, dem Geschlecht und der Gesamtverfassung des Patienten ergeben kann.

Die Therapie eines akuten Lungenödems gemäß der Erfindung erfolgt insofern symptomatisch und effektorientiert. Die Häufigkeit der Gabe der chemisch modifizierten, hoch-molekular vernetzten Hämoglobine beträgt wie erläutert zwischen einmal und einem beliebigen erfolgsabhängigen Höchstwert. Eine Mehrfachgabe kann dabei planmäßig oder bedarfsgesteuert, regelmäßig oder unregelmäßig erfolgen. Die Einzeldosis richtet sich nach der gewünschten therapeutischen Konzentration im Blutplasma und beachtet bereits (oder noch) vorhandene Hämoglobin-Hyperpolymere in diesem Körperkompartiment, dergestalt, dass ein aus anderen Gründen, insbesondere der Erhöhung der Viskosität des Blutplasmas, bereits wieder unerwünschter Höchstwert der Konzentration der Hämoglobin-Hyperpolymeren im Blutplasma von etwa 50 bis 60 g/L nur unter Beachtung des Ergebnisses einer besonders sorgfältigen und kritischen Nutzen-Risiko-Abwägung für den Patienten überschritten wird. Die nach Applikation erreichbare initiale therapeutische Konzentration im Blutplasma ($c_mHb(PL)$) kann aus der verabreichten Dosis der Hämoglobin-Hyperpolymere (mHb) und dem Volumenanteil der Erythrozyten im Blut (dem Hämatokrit: Hkt) und dem Körpergewicht des Patienten (KG) abgeschätzt werden gemäß

$$c_mHb(PL) \quad = \quad mHb \cdot ( BV \cdot KG \cdot (1 - Hkt) )^{-1}$$

wobei als mittlerer Wert für das Blutvolumen (BV) für Frauen 60,5 mL / kg (KG) (57 ... 64 mL / kg (KG)) und für Männer 69,5 mL / kg (KG) (69 ... 70 mL / kg (KG) einzusetzen sind.

Herstellung des erfindungsgemäß zu verwendenden Mittels

Das verwendete Mittel wird hergestellt durch einfaches Einbringen des oder der geeigneten Hämoglobin-Hyperpolymeren in wässrige, insbesondere wässrige (sterile) Elektrolytlösungen, welche den oder die Elektrolyten in der genannten Menge enthalten. Die Hyperpolymeren liegen molekular-dispers vor und können sofort, insbesondere als Injektionen wie beschrieben verabreicht werden.

Beispiele

Die Erfindung wird anhand der folgenden Beispiele näher erläutert. Dabei zeigen die Figuren 1-2 folgendes:

Figur 1 zeigt beispielhaft die Abhängigkeit des onkotischen Druckes ($\pi_{onk}$) einer Lösung eines erfindungsgemäß zur verbesserten Therapie eines akuten Lungenödem verwendeten chemisch modifizierten, hoch-molekular vernetzten Hämoglobins (ein $HP_3Hb$ (pegylierte Schweinehämoglobin-Hyperpolymere), Charge MR A-A) über dessen Massenkonzentration ($c_mHb$) in einer wässrigen Kochsalzlösung der Konzentration 80 g/L.
Figur 2 zeigt die Wirksamkeit chemisch modifizierten, hoch-molekular vernetzten Hämoglobins (beispielhaft gezeigt für ein $HP_3Hb$, Charge MR A-A) zur verbesserten Therapie eines akuten Lungenödem, hier als die Überlebenszeit von zehn narkotisierten Ratten nach Beibringung eines letalen toxischen Lungenödems (durch Injektion von Ölsäure), von denen fünf Tiere zur Therapie den Wirkstoff in ihr Blut addiert bekamen.

Folgende Materialien kamen zum Einsatz:

1. Das chemisch modifizierte, hoch-molekular vernetzte Hämoglobin war ein pegyliertes Schweinehämoglobin-Hyperpolymere (ein $HP_3Hb$, Charge MR A-A), das prinzipiell entsprechend der DE (OS) 100 31 740 A1 aseptisch (im Labormaßstab) hergestellt wurde. Speziell wurde die Charge MR A-A aus einer Mischung der Syntheseprodukte-Chargen MR 14, MR 15 und MR 16 durch präparative Ultrafiltration gewonnen.
MR 14: Steriles, hochreines Schweinehämoglobin, in einer Konzentration von 289 g/L gelöst in einem wässrigen Elektrolyten der Zusammensetzung 20 mM $NaHCO_3$ und 150 mM NaCl, wurde bei 4 °C durch Rühren der Lösung unter ständig erneuertem, reinen Stickstoff desoxygeniert, 4 mol Natrium-Ascorbat (als 1-molare Lösung in Wasser) je Mol Hämoglobin zugegeben und etwa 15 Stunden reagieren lassen, die Lösung mit 2-molarer Milchsäure auf einen pH-Wert von 5,7 titriert, 2 Mol Inositolhexaphosphat (als 0,25-molare Lösung in Wasser) pro mol Hämoglobin zugegeben, nach etwa 1 Stunde mit 2-molarer Milchsäure auf einen pH-Wert von 6,5 titriert, 9,9 Mol Glutardialdehyd (als etwa 1,9-%-ige Lösung in deoxygeniertem Wasser) je Mol Hämoglobin zur Vernetzung des Hämoglobins innerhalb 1,5 Stunden zugegeben, 1,8 L Wasser, das mit Stickstoff äquilibriert war, je Liter initiale Hämoglobinlösung zugegeben, nach 20 Stunden mit 0,5-molarer Natronlauge auf einen pH-Wert von 6,9 titriert, 20 Mol Natriumborhydrid (als 1-molare Lösung in 0,01-molarer Natronlauge) je Mol Hämoglobin zugegeben und für 15 Minuten reagieren lassen, 4 Mol Methoxy-Succinimidylpropionat-Polyethylenglykol des Molekulargewichts 1000 g/mol (als etwa 25-%-ige Lösung in Wasser) zugegeben und für 1 Stunde reagieren lassen, und schließlich die Stickstoffatmosphäre durch reinen Sauerstoff ersetzt und eine Stunde lang äquilibrieren lassen. Ungelöste Bestandteile wurden durch Zentrifugation (10 min mit 20.000 g) abgetrennt und die überstehende Lösung zur weiteren Klärung durch Filter abnehmender Porenweite, zuletzt von 0,2 $\mu$m, filtriert.

MR 15: Steriles, hochreines Schweinehämoglobin, in einer Konzentration von 281 g/L gelöst in einem wässrigen Elektrolyten der Zusammensetzung 20 mM NaHCO$_3$ und 150 mM NaCl, wurde bei 4 °C durch Rühren der Lösung unter ständig erneuertem, reinen Stickstoff desoxygeniert, 4 mol Natrium-Ascorbat (als 1-molare Lösung in Wasser) je Mol Hämoglobin zugegeben und etwa 3 Stunden reagieren lassen, die Lösung mit 2-molarer Milchsäure auf einen pH-Wert von 5,7 titriert, 2 Mol Inositolhexaphosphat (als 0,25-molare Lösung in Wasser) pro mol Hämoglobin zugegeben, nach etwa 1 Stunde mit 2-molarer Milchsäure auf einen pH-Wert von 6,3 titriert, 9,9 Mol Glutardialdehyd (als etwa 1,9-%-ige Lösung in deoxygeniertem Wasser) je Mol Hämoglobin zur Vernetzung des Hämoglobins innerhalb 1,5 Stunden zugegeben, 1,8 Liter Wasser, das mit Stickstoff äquilibriert war, je Liter initiale Hämoglobinlösung zugegeben, nach 17 Stunden mit 0,5-molarer Natronlauge auf einen pH-Wert von 6,9 titriert, 20 Mol Natriumborhydrid (als 1-molare Lösung in 0,01-molarer Natronlauge) je Mol Hämoglobin zugegeben und für 15 Minuten reagieren lassen, 4 Mol Methoxy-Succinimidylpropionat-Polyethylenglykol des Molekulargewichts 1000 g/mol (als etwa 25-%-ige Lösung in Wasser) zugegeben und für 1 Stunde reagieren lassen, und schließlich die Stickstoffatmosphäre durch reinen Sauerstoff ersetzt und eine Stunde lang äquilibrieren lassen. Ungelöste Bestandteile wurden durch Zentrifugation (10 min mit 20.000 g) abgetrennt und die überstehende Lösung zur weiteren Klärung durch Filter abnehmender Porenweite, zuletzt von 0,2 $\mu$m, filtriert.

MR 16: Steriles, hochreines Schweinehämoglobin, in einer Konzentration von 262 g/L gelöst in einem wässrigen Elektrolyten der Zusammensetzung 20 mM NaHCO$_3$ und 150 mM NaCl, wurde bei 4 °C durch Rühren der Lösung unter ständig erneuertem, reinen Stickstoff desoxygeniert, 4 mol Natrium-Ascorbat (als 1-molare Lösung in Wasser) je Mol Hämoglobin zugegeben und etwa 27 Stunden reagieren lassen, die Lösung mit 2-molarer Milchsäure auf einen pH-Wert von 5,8 titriert, 2 Mol Inositolhexaphosphat (als 0,25-molare Lösung in Wasser) pro mol Hämoglobin zugegeben, nach etwa 1,5 Stunde mit 2-molarer Milchsäure auf einen pH-Wert von 6,5 titriert, 9,9 Mol Glutardialdehyd (als etwa 1,9-%-ige Lösung in deoxygeniertem Wasser) je Mol Hämoglobin zur Vernetzung des Hämoglobins innerhalb 1,5 Stunden zugegeben, 1,8 Liter Wasser, das mit Stickstoff äquilibriert war, je Liter initiale Hämoglobinlösung zugegeben, nach 17 Stunden mit 0,5-molarer Natronlauge auf einen pH-Wert von 6,9 titriert, 20 Mol Natriumborhydrid (als 1-molare Lösung in 0,01-molarer Natronlauge) je Mol Hämoglobin zugegeben und für 1,5 Stunden reagieren lassen, 4 Mol Methoxy-Succinimidylpropionat-Polyethylenglykol des Molekulargewichts 1000 g/mol (als etwa 25-%-ige Lösung in Wasser) zugegeben und für 1 Stunde reagieren lassen, und schließlich die Stickstoffatmosphäre durch reinen Sauerstoff ersetzt und eine Stunde lang äquilibrieren lassen. Ungelöste Bestandteile wurden durch Zentrifugation (10 min mit 20.000 g) abgetrennt und die überstehende Lösung zur weiteren Klärung durch Filter abnehmender Porenweite, zuletzt von 0,2 $\mu$m, filtriert.

MR A-A: 3720 mL MR 14 mit 107 g Hämoglobin-Polymeren, 3600 mL MR 15 mit 115 g Hämoglobin-Polymeren und 3900 mL MR 16 mit 127 g Hämoglobin-Polymeren wurden gemischt und in mehreren Anteilen in einer Ultrafiltrationsanlage (Centramate von Pall-Filtron) bei einer mittleren Konzentration von 40 g/L über und durch Celluloseacetatmembranen der Nominellen Molmassen-Trenngrenze 1 MDa fraktioniert, wobei der Filtratfluss durch ein Ventil auf Werte unter 50 % des Maximums (der so genannter Wasserfluss) eingestellt war und jeweils mindestens das zehnfache Volumen der Vorlage an Diafiltrationslösung (diese enthielt Kochsalz in einer Konzentration von 80 g/L) in einem kontinuierlichen Diafiltrationsmodus zum gleichzeitigen Lösungsmitteltausch verwendet wurden. Zum Abschluss wurden die Retentate konzentriert und später vereinigt.

Diese präparativ abgetrennte Fraktion des Wirkstoffes besaß eine Molmassenverteilung mit einem Zahlenmittel der Molmassen von 230.000 g/mol und einem Massenmittel von 993.000 g/mol. Der so erhaltene Wirkstoff kam in einer sterilen und gemäß Ph. Eur. hinreichend endotoxinarmen wässrigen Lösung von 80 g/L NaCl in WFI (Wasser für Injektionszwecke) zum Einsatz. Sein Massengehalt betrug 58 g/L, der pH-Wert der Zubereitung 7,3.

2. Die Versuchstiere waren weiße Laborratten mit einem mittleren Körpergewicht von etwa 350 g (die Spannweite der Körpergewichte aller verwendeten zehn Tiere betrug zwischen 315 und 390 g), die gemäß gültiger Tierschutzregeln gezüchtet und gehalten waren. Sie hatten bis zum Vortag des jeweiligen Experiments freien Zugang zu hinreichender Nahrung, trinken konnten sie bis unmittelbar vor Versuchsbeginn.

Folgende speziellen Bestimmungsmethoden wurden angewendet:

1. Hämoglobingehalte wurden fotometrisch mit der modifizierten Cyanhämoglobin-Methode nach Drabkin (‚Hämoglobin-Farbtest MRP3', Boehringer Mannheim, D), pH-Werte potentiometrisch (pH-Glaselektrode) mit einem Blutgasanalysator (‚ABL 5', Radiometer, Willich, D) gemessen.

2. Bestimmungen der Molekulargewichts-Verteilungen der vernetzten Hämoglobine, sowie charakteristischer Kennwerte dieser erfolgten durch Volumenausschluss-Chromatografie (gemäß Pötzschke H. et al. (1997): "Molar Masses and Structure in Solution of Haemoglobin Hyperpolymers - A Common Calibration of Size Exclusion Chromatography of These Artificial Oxygen Carriers", Artificial Cells, Blood Substitutes, and Immobilization Biotechnology 25, 527 - 540) am Gel Sephacryl S-400 HR (Pharmacia Biotech, Freiburg, D).

3. Bestimmungen der onkotischen Drücke wässriger Lösungen der vernetzten Hämoglobine erfolgten mit Membranosmometem (Membrane-Osmometer oder Colloid-Osmometer, Knauer, Berlin, D), einer wässrigen Kochsalz-Lösung (80 g/L NaCl und 0,2 g/L $NaN_3$) als Lösungsmittel und CelluloseacetatMembranen der Nominellen Molmassen-Trenngrenze 20.000 Da.

Ausführungsbeispiel1: Vergleichsgruppe

Fünf Laborratten bekamen eine systemische Vollnarkose durch intraperitoneale Injektion von 50 mg Pentobarbital je kg Körpergewicht. Die Narkosetiefe wurde über die Zeit konstant im (chirurgischen) Toleranzstadium (dem 3. klassischen Narkosestadium nach A. Guedel) gehalten, indem jeweils nach Auftreten einer aktiven Schmerzreaktion (Zurückziehen) auf ein periodisches Kneifen einer Pfote erneut 17 mg Pentobarbital je kg Körpergewicht als Erhaltungsdosis verabreicht wurden (in etwa alle 90 min). Die Tiere erhielten einen venösen Katheter aus PE-Schlauch in eine Vena jugularis implantiert, und im weiteren Verlauf der Versuche je Stunde etwa 0,5 mL einer isotonen Kochsalzlösung (90 g NaCl je L Lösung) zur Aufrechterhaltung einer ausgeglichenen Flüssigkeitsbilanz über diesen Katheter intravenös verabreicht.

Den Tieren wurde, zur Erzeugung eines akuten toxischen Lungenödems, genau 48 $\mu$L Ölsäure je kg Körpergewicht über drei Minuten gleichmäßig intravenös verabreicht.

Abbildung 2 zeigt im linkem Teil die Überlebenszeiten der fünf Tiere, die alle in weniger als vier Stunden verstarben, vier von ihnen sogar in weniger als drei Stunden (gerechnet ab der Injektion der Ölsäure)

Ausführungsbeispiel 2: Therapiegruppe

Fünf weitere Ratten wurden ansonsten ganz analog wie im Ausführungsbeispiel 1 beschrieben behandelt, mit dem einzigen Unterschied, dass ihnen 15 und 45 min nach der intravenösen Gabe der Ölsäure jeweils 2,5 mL der Zubereitung des chemisch modifizierten, hoch-molekular vernetzten Hämoglobins (ein $HP_3Hb$, ein pegyliertes Schweinehämoglobin-Hyperpolymere - Charge MR A-A) intravenös verabreicht wurde. Der mittlere Hämoglobingehalt im Blutplasma nach der zweiten Gabe betrug etwa 23 g/L, der anschließend langsam (mit einer Plasmahalbwertszeit von etwa 18 Stunden) reduziert wurde.

Abbildung 2 zeigt im rechten Teil die Überlebenszeiten dieser fünf Tiere, die ausnahmslos länger als sieben Stunden überlebten und schließlich, um die Versuche zu beenden, in Narkose getötet wurden.

Der Vergleich der Überlebenszeiten der Tiere in beiden Experimentalgruppen zeigt die enorme Wirksamkeit der Hämoglobin-Hyperpolymeren zur Verminderung der spontanen Letalität hier experimentell induzierter toxischer Lungenödeme.

## Patentansprüche

1. Verwendung wässriger hypo-onkotischer Lösungen, enthaltend Elektrolyte und chemisch modifizierte, hoch-molekular vernetzte hyperpolymere Hämoglobine, zur Herstellung eines Mittels zur Behandlung akuter Lungenödeme.

2. Verwendung gemäß Anspruch 1, **dadurch gekennzeichnet, dass** die chemisch modifizierten hyperpolymeren Hämoglobine vom Menschen, vom Schwein oder vom Rind stammen.

3. Verwendung gemäß einem der Ansprüche 1 oder 2, **dadurch gekennzeichnet, dass** der onkotische Druck in den wässrigen Elektrolytlösungen weniger als 5 mbar beträgt.

4. Verwendung gemäß einem der Ansprüche 1 bis 3, **dadurch gekennzeichnet, dass** Kochsalz in einer Konzentration zwischen 50 und 150 g/L enthalten ist.

5. Verwendung gemäß einem der Ansprüche 1 bis 4, **dadurch gekennzeichnet, dass** Elektrolyte, entsprechend den physiologischem Milieu enthalten sind.

6. Verwendung gemäß einem der Ansprüche 1 bis 5, **dadurch gekennzeichnet, dass** an die modifizierten hyperpolymeren Hämoglobine ein Polyalkylenoxid, kovalent gebunden ist.

7. Verwendung gemäß einem der Ansprüche 1 bis 6, **dadurch gekennzeichnet, dass** das Mittel in Form der wässrigen Lösung als Injektion intravasal verabreicht wird.

8. Verwendung gemäß einem der Ansprüche 1 bis 7, **dadurch gekennzeichnet, dass** das Mittel in Form der wässrigen Lösung ein oder mehrmals verabreicht wird.

**Claims**

1. Use of aqueous hypo-oncotic solutions containing electrolytes and chemically modified, high molecular weight crosslinked hyperpolymeric hemoglobins for preparing an agent for treating acute pulmonary oedema.

2. The use according to claim 1, **characterized in that** the chemically modified hyperpolymeric hemoglobins originate from humans, pigs, or cattle.

3. The use according to claim 1 or 2, **characterized in that** the oncotic pressure in the aqueous electrolyte solutions is below 5 mbars.

4. The use according to any of claims 1 to 3, **characterized in that** sodium chloride is contained at a concentration between 50 and 150 g/L.

5. The use according to any of claims 1 to 4, **characterized in that** electrolytes are contained corresponding to the physiological medium.

6. The use according to any of claims 1 to 5, **characterized in that** a polyalkylene oxide is covalently bonded to the modified hyperpolymeric hemoglobins.

7. The use according to any of claims 1 to 6, **characterized in that** the agent is administered in the form of the aqueous solution by intravascular injection.

8. The use according to any of claims 1 to 7, **characterized in that** the agent is administered in the form of the aqueous solution one or more times.

**Revendications**

1. Utilisation de solutions aqueuses hypooncotiques, contenant des électrolytes et des hémoglobines chimiquement modifiées, de haut poids moléculaire réticulées hyperpolymères pour la préparation d'un agent pour le traitement d'oedèmes pulmonaires aigus.

2. Utilisation selon la revendication 1, **caractérisée en ce que** les hémoglobines chimiquement modifiées hyperpolymères proviennent de l'homme, du porc ou du boeuf.

3. Utilisation selon l'une quelconque des revendications 1 ou 2, **caractérisée en ce que** la pression oncotique dans les solutions électrolytiques aqueuses est inférieure à 5 mbars.

4. Utilisation selon l'une quelconque des revendications 1 à 3, **caractérisée en ce que** du chlorure de sodium est contenu en une concentration entre 50 et 150 g/l.

5. Utilisation selon l'une quelconque des revendications 1 à 4, **caractérisée en ce que** des électrolytiques correspondant au milieu physiologique sont contenus.

6. Utilisation selon l'une quelconque des revendications 1 à 5, **caractérisée en ce qu'**un poly(oxyde d'alkylène) est lié par covalence aux hémoglobines modifiées hyperpolymères.

7. Utilisation selon l'une quelconque des revendications 1 à 6, **caractérisée en ce que** l'agent est administré sous forme de solution aqueuse en tant qu'injection par voie intravasculaire.

8. Utilisation selon l'une quelconque des revendications 1 à 7, **caractérisée en ce que** l'agent est administré sous forme de solution aqueuse, une ou plusieurs fois.

**Figur 1**

**Figur 2**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19701037
- EP 97100790 A
- DE 4418973
- DE 3841105
- DE 3714351
- DE 3576651

- DE 10031744 A1
- DE OS10031740 A
- DE OS10031742 A
- DE OS10031744 A1
- DE 10031740 A1
- DE OS10031740 A1

**In der Beschreibung aufgeführte Nicht-Patentliteratur**

- **RIESS J. G.** Oxygen Carriers (''Blood Substitutes'') - Raison d'Etre, Chemistry, and Some Physiology. *Chemical Reviews,* 2001, vol. 101, 2797-2919
- **VANDEGRIFF K. D.** Haemoglobin-based Oxygen Carriers. *Expert Opinions on Investigational Drugs,* 2000, vol. 9, 1967-1984
- **BARNIKOL W. K. R. et al.** Hyperpolymere Hämoglobine als künstliche Sauerstoffträger - Ein innovativer Ansatz der medizinischen Entwicklung. *Therapiewoche,* 1996, vol. 46, 811-815
- **BÖCKER, W ; DENK, H. ; HEITZ PH. U.** *Pathologie, Urban & Schwarzenberg,* 1997
- **GEROCK W. HUBER CH ; MEINERTZ T ; ZEIDLER H.** Gross = Schölmerich = Gerock - Die Innere Medizin. 2000

- **WEIKRAUCH T.R.** Wolff = Weikrauch - Internistische Therapie. 2000
- **JENA.** *Arch. Cardiol. Mex.,* 2000, vol. 72, 280-285
- **KATREN, V.** The Conjugation of Proteins With Polyethylene Glycol and Other Polymers - Altering Properties of Proteins to Enhance Their Therapeutic Potential. *Advanced Drug Delivery Reviews,* 1993, vol. 10, 91-114
- **PÖTZSCHKE H. et al.** Molar Masses and Structure in Solution of Haemoglobin Hyperpolymers - A Common Calibration of Size Exclusion Chromatography of These Artificial Oxygen Carriers. *Artificial Cells, Blood Substitutes, and Immobilization Biotechnology,* 1997, vol. 25, 527-540